# EUROPÄISCHE PATENTANMELDUNG

(11) **EP 1 207 151 A1**
(43) Veröffentlichungstag der Anmeldung: **22.05.2002**
(21) Anmeldenummer: 01124429.0
(22) Anmeldetag: 11.10.2001
(51) Int. Cl.: C07C 249/02

(54) **Verfahren zur Herstellung von N-Methylenglycinaten**

(30) Priorität: 15.11.2000 DE 10056468
(71) Anmelder: Degussa AG, 40474 Düsseldorf (DE)
(72) Erfinder: Eils, Stefan, Dr., 63450 Hanau (DE); Rossen, Kai, Dr., 63452 Hanau (DE); Jahn, Wilfried, 63571 Gelnhausen (DE); Klement, Ingo, Dr., 35415 Pohlheim-Garbenteich (DE)

(57) **Zusammenfassung**

Die vorliegende Erfindung ist auf ein Verfahren zur Herstellung der Verbindungen der Formel (I) gerichtet.

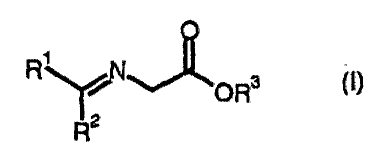

Das Verfahren bedient sich dabei der nukleophilen Substitution von entsprechenden Essigsäurederivaten der allgemeinen Formel (III), 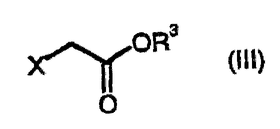 mit Iminen der allgemeinen Formel (II),

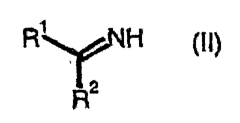

In den Formeln (I)-(III) haben R', R², R³ und X die in der Beschreibung angegebenen Bedeutungen.

## Beschreibung

Die vorliegende Erfindung ist auf ein Verfahren zur Herstellung von N-Methylenglycinaten gerichtet. Insbesondere werden Glycinate der allgemeinen Formel (I) hergestellt.

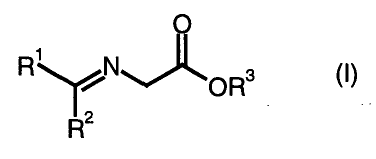

Derartige Substrate sind wichtige Intermediate in der organischen chemischen Synthese und finden Anwendung bei der Herstellung biologisch aktiver Substanzen.

Aus dem Stand der Technik sind mehrere Verfahren zur Herstellung der gewünschten Verbindungen bekannt. So arbeiteten Ibata et al. (Chem. Lett. 1994, 1, 81-84) mit 2-Diazo-essigsäure, welches unter Kupferkatalyse mit Benzophenonimin umgesetzt wurde. Ausgehend von Glycinestern wurde die Umsetzung mit Benzophenonimin untersucht (Fast et al. J. Chem. Soc. Perkin Trans. 1, 1988, 3081-3084; Yaozhong et al. Synth. Commun. 1989, 19, 881-888). Auch Glycinesterhydrochloride bzw. -tosylate wurde mit Benzophenonimin erfolgreich umgesetzt (O'Donnell et al., J. Org. Chem., 1982, 47, 13, 2663-2666).
Weiterhin ist bekannt, daß α-Triflat-substituierte Essigsäureethylester mit N-alkyliertem Benzophenonimim umgesetzt werden können (Vedejs et al. J. Org. Chem. 1987, 52, 3470-3474). Auch Amide der Essigsäure wurden derart transformiert (Moenius et al. J. Labbelled Compd. Radiopharm., 1999, 42, 1006-7).

Aufgabe der vorliegenden Erfindung war die Angabe eines weiteren Verfahrens zur Herstellung von N-Methylenglycinaten.

Diese Aufgabe wird durch ein Verfahren mit den Merkmalen des Anspruchs 1 gelöst. Die von Anspruch 1 abhängigen Untersprüche richten sich auf bevorzugte Ausgestaltungen des erfindungsgemäßen Verfahrens.

Dadurch, daß man in Verfahren zur Herstellung von N-Methylenglycinaten der allgemeinen Formel I, 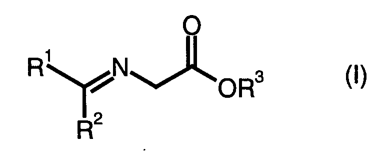 worin
R¹ und R² unabhängig voneinander bedeuten (C₁-C₈)-Alkyl, (C₂-C₈)-Alkoxyalkyl, (C₆-C₁₈)-Aryl, (C₇-C₁₉)-Aralkyl,
(C₃-C₁₈)-Heteroaryl, (C₄-C₁₉)-Heteroaralkyl,
(C₁-C₈)-Alkyl-(C₆-C₁₈)-Aryl,
(C₁-C₈)-Alkyl-(C₃-C₁₈)-Heteroaryl, (C₃-C₈)-Cycloalkyl,
(C₁-C₈)-Alkyl-(C₃-C₈)-Cycloalkyl,
(C₃-C₈)-Cycloalkyl-(C₁-C₈)-Alkyl,
R³ bedeutet (C₁-C₈)-Alkyl, (C₂-C₈)-Alkoxyalkyl,
(C₆-C₁₈)-Aryl, (C₇-C₁₉)-Aralkyl, (C₃-C₈)-Cycloalkyl,
(C₁-C₈)-Alkyl-(C₃-C₈)-Cycloalkyl,
Iminen der allgemeinen Formel II, 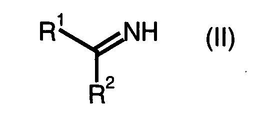 worin R¹ und R² die oben angegebene Bedeutung annehmen, mit Verbindungen der allgemeinen Formel (III), 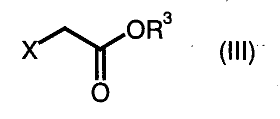 worin R³ die oben angegebene Bedeutung annimmt,
X eine nukleofuge Abgangsgruppe darstellt, umsetzt, gelangt man überraschend einfach, dafür aber nicht minder vorteilhaft in sehr guten Ausbeuten und Reinheit zu den gewünschten Verbindungen. Der erfindungsgemäße Prozeß ist äußerst robust und eignet sich daher besonders gut für den Einsatz im technischen Maßstab. Außerdem sind die Ausgangsmaterialien relativ günstig, so daß der erfindungsgemäße Prozeß auch ökonomisch vorteilhaft erscheint.

Prinzipiell kann X jede für den gegenständlichen Prozeß dem Fachmann geläufige Gruppe sein. Derartige Fluchtgruppen sind in der Literatur bekannt (Advanced Organic Chemistry, J. March, John Wiley and Sons, 4. Ed., 1992, Chapter 10). Bevorzugt ist jedoch ein Verfahren bei dem X ein Rest der Gruppe Halogenid, Alkyl- oder Arylsulfonat oder Carboxylat, insbesondere Chlorid, Bromid, Triflat, Mesylat, p-Tosylat, Trifluorcarboxylat, p-Nitrobenzoat oder Acetat, ist.

Weiter bevorzugt ist ein Verfahren, bei dem die erfindungsgemäße Reaktion in Gegenwart einer Base durchgeführt wird. Hier können alle dem Fachmann bekannten organischen oder anorganischen Basen herangezogen werden. Vorzugsweise handelt es sich jedoch um Trialkylamine, wie Triethylamin, Diisopropylethylamin, oder Carbonate wie Natriumcarbonat, Natriumhydrogencarbonat.

Als Lösungsmittel für die betreffende Reaktion kann jedes dem Fachman für diesen Zweck in Frage kommende organische Lösungsmittel genommen werden, sofern es sich gegenüber der Substitution indifferent verhält. Vorzugsweise handelt es sich um ein polares aprotisches Lösungsmittel. Besonders bevorzugt sind in diesem Zusammenhang Acetonitril, N-Methylpyrrolidon, Dimethylsulfoxid, Dimethylformamid zu nennen.

Die Temperatur der Reaktion sollte so gewählt werden, daß die Reaktionsgeschwindigkeit hinreichend hoch ist, daß Nebenproduktprofil jedoch möglichst günstig ausfällt. Vorzugsweise arbeitet man bei einer Temperatur zwischen 20° - 150°C, insbesondere bei einer Temperatur zwischen 60° - 90°C.

Bei der erfindungsgemäßen Reaktion geht man bevorzugt so vor, daß man den α-X-substituierten Essigsäureester in dem Lösungsmittel der Wahl löst und das Imin hinzugibt. Anschließend wird die Base zum Reaktionsgemisch addiert und dieses unter Rühren erhitzt.
Zur Aufarbeitung neutralisiert man die Reaktionsmischung nach dem Erkalten, vorzugsweise mit einer Carbonsäure wie z.B. Essigsäure, und fügt Wasser hinzu. Anschließend wird die Mischung so weit abgekühlt das ein Niederschlag entstehen kann. Dieser wird abfiltriert und nach Waschen getrocknet.

Das vorliegende Verfahren zur Herstellung dieser relativ komplizierten Iminstrukturen ist äußerst attraktiv für einen großtechnischen Prozeß. So lassen sich die Einsatzstoffe sehr gut recyclieren und zur erneuten Reaktion einsetzen. Damit erhält man ein in ökonomischer wie ökologischer Hinsicht vorteilhaftes Verfahren zur Herstellung der N-Methylenglycinaten.

Als (C₁-C₈)-Alkylreste sind anzusehen Methyl, Ethyl, *n*-Propyl, Isopropyl, *n*-Butyl, Isobutyl, *sec*-Butyl, *tert*-Butyl, Pentyl, Hexyl, Heptyl oder Octyl samt aller ihrer Bindungsisomeren. Der Rest (C₁-C₈)-Alkoxy entspricht dem Rest (C₁-C₈)-Alkyl mit der Maßgabe, daß dieser über ein Sauerstoffatom an das Molekül gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffatome miteinander verbunden sein können. Die Anzahl der Kohlenstoffatome gibt die Gesamtzahl der im Rest enthaltenen Kohlenstoffatome an.

Die eben beschriebenen Reste können einfach oder mehrfach mit Halogenen und/oder N-, O-, P-, S-, Si-atomhaltigen Resten substituiert sein. Dies sind insbesondere Alkylreste der oben genannten Art, welche eines oder mehrere dieser Heteroatome in ihrer Kette aufweisen bzw. welche über eines dieser Heteroatome an das Molekül gebunden sind.

Unter (C₃-C₈)-Cycloalkyl versteht man Cyclopropyl, Cyclobutyl, Cyclopentyl, Cyclohexyl bzw. Cycloheptylreste etc. Diese können mit einem oder mehreren Halogenen und/oder N-, O-, P-, S-, Si-atomhaltigen Resten substituiert sein und/oder N-, O-, P-, S-Atome im Ring aufweisen, wie z. B. 1-, 2-, 3-, 4-Piperidyl, 1-, 2-, 3-Pyrrolidinyl, 2-, 3-Tetrahydrofuryl, 2-, 3-, 4-Morpholinyl.

Ein (C₃-C₈)Cycloalkyl-(C₁-C₈)-Alkylrest bezeichnet einen wie oben dargestellten Cycloalkylrest, welcher über einen wie oben angegebenen Alkylrest an das Molekül gebunden ist.

(C₁-C₈)-Acyloxy bedeutet im Rahmen der Erfindung einen wie oben definierten Alkylrest mit max. 8 C-Atomen, welcher über eine COO-Funktion an das Molekül gebunden ist.

(C₁-C₈)-Acyl bedeutet im Rahmen der Erfindung einen wie oben definierten Alkylrest mit max. 8 C-Atomen, welcher über eine CO-Funktion an das Molekül gebunden ist.

Unter einem (C₆-C₁₈)-Arylrest wird ein aromatischer Rest mit 6 bis 18 C-Atomen verstanden. Insbesondere zählen hierzu Verbindungen wie Phenyl-, Naphthyl-, Anthryl-, Phenanthryl-, Biphenylreste oder an das betreffende Molekül annelierte Systeme der vorbeschriebenen Art, welche ggf. mit (C₁-C₈)-Alkyl, (C₁-C₈)-Alkoxy, (C₁-C₈)-Acyl, (C₁-C₈)-Acyloxy, NO₂, NR¹R², SR¹, SH, SOR¹, SO₂R¹, Hal substituiert sein können.

Ein (C₇-C₁₉)-Aralkylrest ist ein über einen
(C₁-C₈)-Alkylrest an das Molekül gebundener
(C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z. B. Stickstoff, Sauerstoff oder Schwefel im Ring aufweist. Als solche Heteroaromaten werden insbesondere Reste angesehen, wie 1-, 2-, 3-Furyl, wie 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl.

Unter einem (C₄-C₁₉)-Heteroaralkyl wird ein dem (C₇-C₁₉)-Aralkylrest entsprechendes heteroaromatisches System verstanden.

Als Halogene (Hal) kommen Fluor, Chlor, Brom und Iod in Frage. Halogenid bezeichnet die entsprechende ionische Form.

### Herstellvorschriften

### Beispiel 1

3,90 g tert.-Butyl-2-brom-acetat werden in 20 ml Acetonitril gelöst und 3,62 g Benzophenonimin, sowie 2,58 g Diisopropylethylamin zugegeben. Die Lösung wird unter Rühren für 8 h zum Rückfluß erhitzt. Anschließend wird die Reaktionslösung mit 50 % wäßr. Essigsäure bei Raumtemperatur neutralisiert und zusätzlich 30 ml Wasser zugefügt. Beim Kühlen im Eisbad fällt das Produkt aus und wird über eine Fritte abgesaugt. Der Niederschlag wird mit wenig kaltem 90 % Ethanol gewaschen, trockengesaugt und im Vakuum über Phosphorpentoxid getrocknet. Es werden so 4,45 g (75 % d. Th.) tert.-Butyl-N-(diphenylmethylen)glycinat erhalten (Smp. 114,5 °C).

### Beispiel 2

3, 62 g Tert.-butyl-2-chlor-acetat werden in 20 ml N-Methylpyrrolidinon gelöst und 0,6 g Kaliumjodid, 3,32 g Kaliumcarbonat, sowie 3,61 g Benzophenonimin zugefügt. Das Reaktionsgemisch wird für 6 h bei 80 °C gerührt und anschließend auf Raumtemperatur abgekühlt. Es werden je 20 ml Ethanol und Wasser zugetropft. Die basische Lösung wird durch Zugabe von 8 ml Essigsäure auf pH 5,5 gebracht. Nach Zugabe von weiteren 5 ml Ethanol und 30 ml Wasser fällt beim Kühlen im Eisbad das Produkt aus. Es wird über eine Fritte abgesaugt, mit 70 % Ethanol gewaschen und im Vakuum getrocknet. So werden 4,0 g (68 % d. Th.) tert.-Butyl-N-(diphenylmethylen)glycinat erhalten.

## Patentansprüche

1. Verfahren zur Herstellung von N-Methylenglycinaten der allgemeinen Formel I, 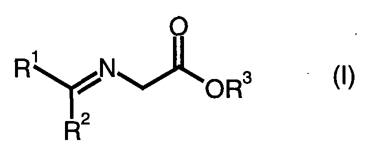 worin
R¹ und R² unabhängig voneinander bedeuten (C₁-C₈)-Alkyl, (C₂-C₈)-Alkoxyalkyl, (C₆-C₁₈)-Aryl,
(C₇-C₁₉)-Aralkyl, (C₃-C₁₈)-Heteroaryl,
(C₄-C₁₉)-Heteroaralkyl, (C₁-C₈)-Alkyl-(C₆-C₁₈)-Aryl,
(C₁-C₈)-Alkyl-(C₃-C₁₈)-Heteroaryl, (C₃-C₈)-Cycloalkyl,
(C₁-C₈)-Alkyl-(C₃-C₈)-Cycloalkyl,
(C₃-C₈)-Cycloalkyl-(C₁-C₈)-Alkyl,
R³ bedeutet (C₁-C₈)-Alkyl, (C₂-C₈)-Alkoxyalkyl,
(C₆-C₁₈)-Aryl, (C₇-C₁₉)-Aralkyl, (C₃-C₈)-Cycloalkyl,
(C₁-C₈)-Alkyl-(C₃-C₈)-Cycloalkyl,
durch Umsetzung von Iminen der allgemeinen Formel II, 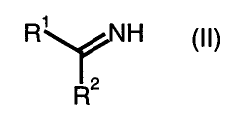 worin R¹ und R² die oben angegebene Bedeutung annehmen,
mit Verbindungen der allgemeinen Formel (III), 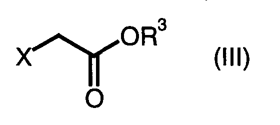 worin R³ die oben angegebene Bedeutung annimmt,
X eine nukleofuge Abgangsgruppe darstellt.

2. Verfahren nach Anspruch 1,
**dadurch gekennzeichnet, daß**
X ein Rest der Gruppe Halogenid, Alkyl- oder Arylsulfonat oder Carboxylat, insbesondere Chlorid, Bromid, Triflat, Mesylat, p-Tosylat, Trifluorcarboxylat, p-Nitrobenzoat, Acetat, ist.

3. Verfahren nach einem oder mehreren der vorhergehenden Ansprüche,
**dadurch gekennzeichnet, daß** man
die Reaktion in Gegenwart einer Base durchführt.

4. Verfahren nach einem oder mehreren der vorhergehenden Ansprüche,
**dadurch gekennzeichnet, daß** man
die Reaktion in einem indifferenten, insbesondere einem polaren, organischen Lösungsmittel durchführt.

5. Verfahren nach einem oder mehreren der vorhergehenden Ansprüche,
**dadurch gekennzeichnet, daß** man
die Reaktion bei einer Temperatur zwischen 20° - 150°C, insbesondere bei einer Temperatur zwischen 60° - 90°C, durchführt.
